(19) Europäisches Patentamt / European Patent Office / Office européen des brevets

(11) **EP 3 998 329 A1**

(12) ## EUROPEAN PATENT APPLICATION
published in accordance with Art. 153(4) EPC

(43) Date of publication:
**18.05.2022 Bulletin 2022/20**

(21) Application number: **20836851.4**

(22) Date of filing: **07.07.2020**

(51) International Patent Classification (IPC):
*C12M 1/00* (2006.01)   *C12M 3/00* (2006.01)

(52) Cooperative Patent Classification (CPC):
**C12M 1/00; C12M 3/00**

(86) International application number:
**PCT/JP2020/026595**

(87) International publication number:
**WO 2021/006278 (14.01.2021 Gazette 2021/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.07.2019   JP 2019127121**

(71) Applicant: **Nipro Corporation**
**Osaka-shi, Osaka 531-8510 (JP)**

(72) Inventors:
• **YOSHIKAWA, Yoshihiro**
  **Osaka-shi, Osaka 531-8510 (JP)**

• **TAKEUCHI, Masakatsu**
  **Osaka-shi, Osaka 531-8510 (JP)**
• **YAO, Masafumi**
  **Osaka-shi, Osaka 531-8510 (JP)**
• **YAMAMOTO, Hideaki**
  **Osaka-shi, Osaka 531-8510 (JP)**
• **WAGATSUMA, Yusuke**
  **Osaka-shi, Osaka 531-8510 (JP)**
• **NAKAMURA, Ryosuke**
  **Osaka-shi, Osaka 531-8510 (JP)**

(74) Representative: **Kuhnen & Wacker**
**Patent- und Rechtsanwaltsbüro PartG mbB**
**Prinz-Ludwig-Straße 40A**
**85354 Freising (DE)**

(54) **MULTILAYER CULTURE VESSEL**

(57)    A multilayer culture vessel, comprising a culture vessel part and a reservoir part attached to the culture vessel part, wherein the culture vessel part comprises at least two culture trays that are stacked; the reservoir part comprises a surrounding wall defining an internal space and a port communicating with the internal space; the surrounding wall comprises a first surrounding wall part provided with the port and a second surrounding wall part facing the first surrounding wall part; the culture vessel part has openings communicating individual culture trays of the at least two culture trays with the internal space of the reservoir part; and the openings of the culture vessel part extend toward a first abutting portion that the first surrounding wall part abuts on the culture vessel part from a second abutting portion that the second surrounding wall part abuts on the culture vessel part.

Figure 1

**Description**

**TECHNICAL FIELD**

The invention relates to a culture vessel for culturing cells. In particular, the invention relates to a multilayer culture vessel. The invention also relates to a method for producing a pharmaceutical composition with the multilayer culture vessel.

**BACKGROUND**

Cells are cultured in various technical fields including pharmaceutical fields relating to cellular medicines and research fields relating to cell science. Cell culturing requires, in general, sterilized culture vessels. Culture vessels are used in sterile environments such as in clean benches. Cell suspension is introduced into the internal space of culture vessel through its port. Ports are covered with caps to keep the internal space sterilized. Culture vessels are placed in culture devices with regulated temperatures for culturing cells. Depending on the condition of cells, culture vessels are transferred to clean benches again to conduct operations such as collecting the cells or changing the culture medium.
Cell culture especially in pharmaceutical fields further requires keeping the inside of cell culture facilities as clean as possible. This requires considerable labor and cost to manage and maintain the sterilized condition. Cell culture vessels capable of culturing cells efficiently are required to effectively utilize the facilities' inside space. For example, culture bags (Patent Literature 1) or multilayer culture flasks in which several flat and hard plates are stacked (Patent Literatures 2 and 3) are used to culture a large number of floating cells or adhesive cells.

**CITATION LIST**

    Patent Literature 1: JP 2006-262876 A
    Patent Literature 2: JP 2011-528226 A
    Patent Literature 3: JP 2009-502165 A

**SUMMARY**

**TECHNICAL PROBLEM**

Cell culturing is affected by culture conditions such as the number of cells plated on culture trays and the amount of culture medium. In multilayer culture vessels, it is hard to change culture medium or to subculture cells on each layer separately. A different culture condition on one of the layers may cause the requirement of changing culture medium or subculturing cells on the tray ahead of the other trays. In that case, culture medium change or cell subculturing is required for all the trays, even if it is not necessary for the other trays. This leads to wasted reagents. Reagents for cell culture are relatively expensive. Waste of reagents brings a relatively large economic disadvantage.
Patent literature 2 describes a multilayer culture vessel with a resealable port arranged such that the port axis and the tray surfaces of the culture trays are perpendicular. When cell suspension is injected into the multilayer culture vessel through its resealable port, the cell suspension mostly flows into the bottom culture tray. The cells in the suspension get to accumulate on the tray during the injection process, so that the cell suspension becomes heterogeneous. After completion of the injection, the cell suspension flowed into the bottom culture tray is distributed to the individual culture trays by tilting the multilayer culture vessel. The distribution operation hardly makes the heterogeneous cell suspension homogeneous, and it is difficult to adjust culture conditions for individual culture trays. One object of the present invention is accordingly to provide a multilayer culture vessel comprising at least two culture trays, in which it is easy to adjust culture conditions for individual culture trays in the multilayer culture vessel.
Patent literature 3 describes a multilayer culture vessel comprising a plurality of culture chambers and tracheal spaces, the chambers being covered with filters, and the chambers and the spaces being alternatively stacked with supports. The culture chambers covered with the filters are provided with a manifold enabling to exhaust air from the culture chambers and to supply culture liquid to the culture chambers. The multilayer culture vessel of patent literature 3 is complicated in structure. A multilayer culture vessel with a simple structure was demanded in the field.
A culture vessel with an easy-to-operate shape is advantageous since cellular medical compositions are produced in devices such as clean benches. A multilayer culture vessel with an easy-to-operate shape is demanded in the field.

## SOLUTION TO PROBLEM

The present invention relates to a multilayer culture vessel and a method for producing a pharmaceutical composition with the multilayer culture vessel, as described below.

[Item 1] A multilayer culture vessel, comprising a culture vessel part and a reservoir part attached to the culture vessel part, wherein the culture vessel part comprises at least two culture trays that are stacked; the reservoir part comprises a surrounding wall defining an internal space and a port communicating with the internal space; the surrounding wall comprises a first surrounding wall part provided with the port and a second surrounding wall part facing the first surrounding wall part; the culture vessel part has openings communicating individual culture trays of the at least two culture trays with the internal space of the reservoir part; and the openings of the culture vessel part extend toward a first abutting portion that the first surrounding wall part abuts on the culture vessel part from a second abutting portion that the second surrounding wall part abuts on the culture vessel part.

[Item 2] A multilayer culture vessel, comprising a culture vessel part and a reservoir part attached to the culture vessel part, wherein the culture vessel part comprises at least two culture trays that are stacked; the reservoir part has an internal space and comprises a port communicating with the internal space; the culture vessel part has openings communicating individual culture trays of the at least two culture trays with the internal space of the reservoir part; and the port is provided in the reservoir part such that an angle formed between an axis of the port and a stacking direction that the at least two trays stack is from not less than 70 degrees to not more than 90 degrees and an angle formed between the axis of the port and a surface that the reservoir part is opposed to the at least two culture trays is from not less than zero degrees to not more than 50 degrees.

[Item 3] A method for producing a pharmaceutical composition, the method comprising: culturing cells with the multilayer culture vessel according to Item 1 or 2; collecting from the multilayer culture vessel cultured cells or a culture fluid containing a component secreted from the cultured cells; and producing the pharmaceutical composition comprising the collected cultured cells, the collected secreted component, or a component isolated and purified from the collected cultured cells.

## EFFECTS OF INVENTION

A multilayer culture vessel comprising at least two culture trays according to an embodiment of the present invention allows the culture conditions to be adjusted for the individual culture trays of the multilayer culture vessel, so that waste of reagents may be suppressed. A multilayer culture vessel of another embodiment of the present invention is convenient owing to a simple and/or easy-to-operate structure.

## BRIEF DESCRIPTION OF DRAWINGS

[FIG. 1] Fig. 1 is an exploded perspective view of a multilayer culture vessel according to Embodiment 1.

[FIG. 2] Fig. 2(a) is a front view, on a reduced scale, of the multilayer culture vessel according to Embodiment 1. Fig. 2(b) is a cross-sectional view, on a reduced scale, taken along the A-A line in Fig. 2(a). Fig. 2(c) is an enlarged view of the part surrounded by the broken lines in Fig. 2(b). Fig. 2(d) is a cross-sectional view, on a reduced scale, taken along the D-D line in Fig. 2 (a).

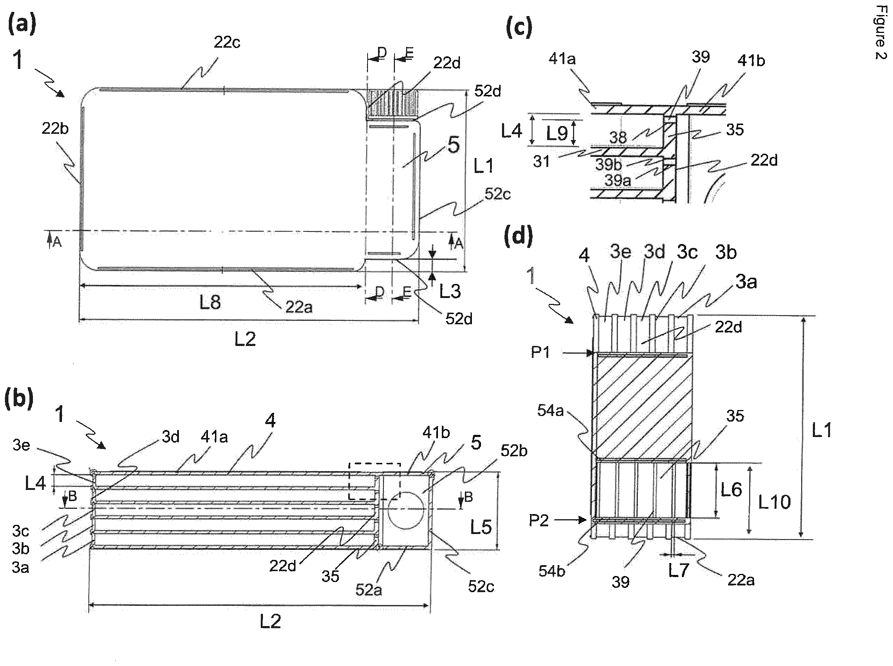

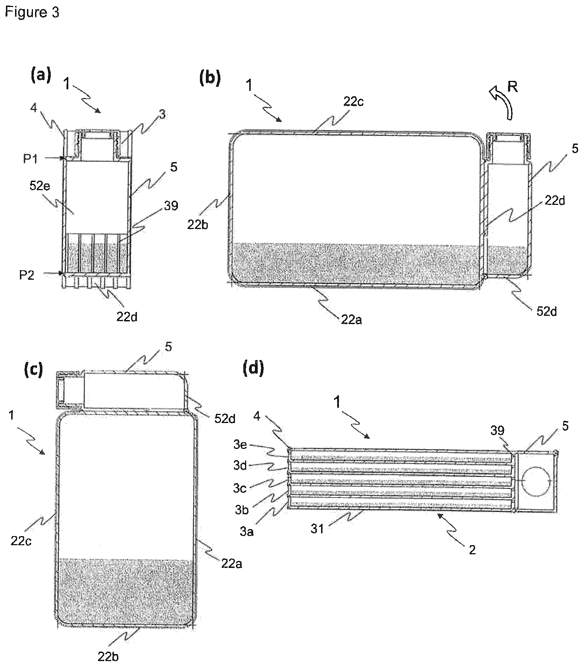

[FIG. 3] Fig. 3 (a) is a cross-sectional view, on a reduced scale, taken along the E-E line in Fig. 2(a) when the multilayer culture vessel is placed in the first posture. Fig. 3 (b) is a cross-sectional view, on a scale view, taken along the B-B line in Fig. 2 (b) when the multilayer culture vessel is placed in the first posture. Fig. 3(c) is a cross-sectional view, on a reduced scale, taken along the B-B line in Fig. 2(b) when the multilayer culture vessel is placed in the second posture. Fig. 3(d) is a cross-sectional view, on a reduced scale, taken along the A-A line in Fig. 2(a) when the multilayer culture vessel is placed in the third posture.

[FIG. 4] Fig. 4 is a perspective view of a multilayer culture vessel according to Embodiment 2.

## DESCRIPTION OF EMBODIMENTS

A "multilayer culture vessel" as used herein means a culture vessel comprising at least two culture trays. Multilayer culture vessels are made, for example, from transparent, translucent, or non-transparent glass or plastic material. The material of the multilayer culture vessels includes, but is not limited to, plastics conventionally used in pharmaceutical or research fields, such as polystyrene, polycarbonate, polyethylene, polypropylene, and polyethylene terephthalate. The multilayer culture vessels may be produced, for example, by assembling a plurality of parts for the vessels. The multilayer culture vessels may be produced according to known methods. For example, multilayer culture vessels may be produced by individually preparing multiple parts by injection molding and assembling the parts.

The parts for assembling the multilayer culture vessel may all be made from the same material. At least one part may be made from a different material. Alternatively, all the parts may be made from different materials. The multilayer culture vessels are, for example, made from the same transparent or translucent material. Individual parts are prepared, for example, to have a thickness of 1-5 mm. The multilayer culture vessels may be, for example, sterilized. Sterilization treatment may be, for example, radiation sterilization, ethylene oxide gas sterilization, $\gamma$-ray sterilization, and high-pressure steam sterilization.

A "culture tray" as used herein means a tray providing a storage space capable of being used as a culture vessel. The storage space of the culture tray is defined by a bottom wall and a surrounding wall surrounding the bottom wall. The bottom wall of the culture tray has a tray surface facing the storage space. The tray surface may be treated for the improvement of cell adhesion. The treatment for improving cell adhesion includes, for example, plasma treatment, oxidant treatment, and coating treatment with a hydrophilic material. The surrounding wall of the culture tray is, for example, integrally molded with the bottom wall, is a wall extending from the outer periphery, is a wall attached to the bottom wall, or is a combination thereof.

A "stacking direction" as used herein means a direction that tray surfaces of the bottom walls of culture trays stack. The stacking direction corresponds to, for example, a direction that the liquid levels of the liquid samples injected into culture trays overlay when the multilayer culture vessel is placed in a posture suitable for culturing. For example, the posture of the multilayer culture vessel suitable for culturing is a posture that the liquid sample injected into the culture tray has a maximum area of liquid surface thereof.

A "port axis" as used herein means an axis extending perpendicular to an opening surface of a port from the geometric center of the opening surface. When the opening surface of the port is circular, the port axis is an axis that passes through the center of the opening surface and extends perpendicular to the opening surface.

A "liquid sample" as used herein means a solvent and a solution. The fluid sample is, for example, culture medium, buffer solution, cell suspension, water, and aqueous solution containing a biochemical reagent such as trypsin. Culture media or buffers are commercially available or can be prepared with known reagents. Cell suspensions are liquids containing cells in cell media or buffers. Biochemical reagents such as trypsin are commercially available or can be prepared according to known methods.

A "pharmaceutical composition" as used herein means a composition including an active ingredient and a pharmaceutically acceptable carrier. Pharmaceutically acceptable carriers may be known carriers and can be appropriately used according to types of active ingredients, administration routes, and dosage forms. Pharmaceutical compositions can be prepared according to known methods. Active ingredients may be, for example, components secreted from cells during culturing, components accumulated in cells, or cells themselves.

"Culturing cells" or "cell culture" as used herein means proliferating cells in multilayer culture vessel or producing in cells components of interest. Cell culturing includes, for example, placing cells in multilayer culture vessels under the environment where temperature, humidity and/or concentration of carbon dioxide are controlled. Cell culturing may further include replacing the medium in which cells were cultured and subculturing cultured cells. The cell includes, for example, established cell line, genetically modified cell, cell obtained from living organism.

"Collecting" as used herein means taking out a liquid sample from a multilayer culture vessel. A liquid sample collected from multilayer culture vessels is, for example, cell suspension or culture medium including cultured cells. The collection from multilayer culture vessels can be appropriately carried out according to known methods. For example, a liquid sample can be taken out from a multilayer culture vessel using a dispensing device such as pipette. A cell suspension including cultured cells can be obtained by, for example, injecting a solution containing a reagent such as trypsin and the like with a dispensing device such as pipette to a multilayer culture vessel in which cells are cultured to release cells adhesive to culture trays. The obtained cell suspension can be taken out from the multilayer culture vessel in the same manner as above.

"Separating" used herein means taking out a component of interest from conditions in which the component exists. For example, a component produced in cultured cells can be separated according to known methods including physical methods such as French press and crushing with ultrasonic wave, or chemical methods such as using a disruption solution containing surfactant or the like.

"Purifying" as used herein means an operation for increasing the content rate of a component of interest. Purification is carried out, for example, according to known methods. The known purification methods include, but are not limited to, liquid chromatography, centrifugation, magnetic beads, and flow cytometer.

Hereinafter, embodiments according to aspects of the present invention will be described with reference to the drawings, but these embodiments are examples of the present invention. The embodiments do not limit the inventions described in the appended claims in any way.

[Embodiment 1]

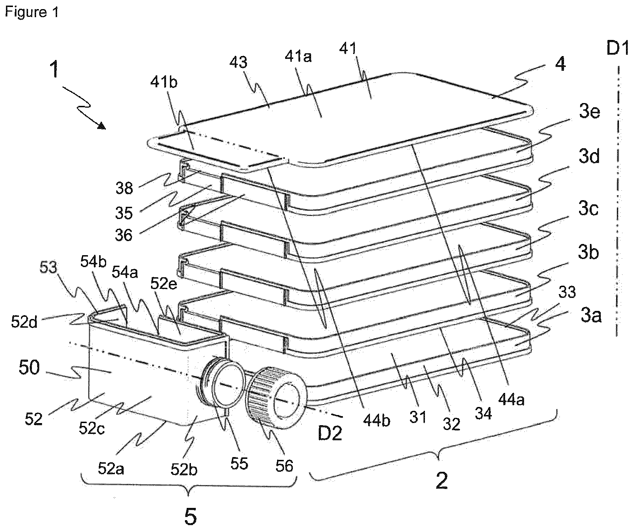

Fig. 1 shows an exploded perspective view of a multilayer culture vessel of an embodiment according to the present invention ("Embodiment 1"). The multilayer culture vessel 1 according to Embodiment 1 comprises a culture vessel part 2 and a reservoir part 5 attached to the culture vessel part 2.

The culture vessel part 2 according to Embodiment 1 constitutes a culture tray 3a, a culture tray 3b stacked on the culture tray 3a, a culture tray 3c stacked on the culture tray 3b, a culture tray 3d stacked on the culture tray 3c, a culture tray 3e stacked on the culture tray 3d, and a cover plate 4 stacked on the culture tray 3e. The reservoir part 5 is attached to the culture vessel part 2, the culture trays 3 and the cover plate 4. The reservoir part 5 has an internal space and a screw port 55 communicating with the internal space. The culture trays 3, the cover plate 4, and the reservoir part 5 are liquid-tightly bonded by heat bond, melt bond, ultrasonic fusion, or adhesive at joint areas or abutting portions described below,

The culture tray 3 includes a bottom wall 31 whose tray surface is substantially rectangular and flat and a surrounding wall 32 that surrounds the bottom wall and is integrally molded with the bottom wall 31. The surrounding wall 32 of the culture tray comprises peripheral wall parts, which extend substantially vertically upward from each periphery of the bottom wall 31 and have a predetermined length. The culture tray 3 has a storage space defined by the bottom wall 31 and the wall 32 surrounding the bottom wall 31. The five culture trays 3a, 3b, 3c, 3d, and 3e have substantially the same shape as each other.

The surrounding wall 32 of the culture tray 3a has a protrusion 33 that facilitates properly stacking with the culture tray 3b. The outer surface of the bottom wall 31 of the culture trays 3b has a recess 34 fitting the protrusion 33 on the surrounding wall 32 of the culture tray 3a. The culture tray 3b can be easily stacked on the culture tray 3a properly by putting the projection 33 of the culture tray 3a into the recess 34 of the culture tray 3b. The outer surfaces of the bottom walls 31 of the culture trays 3c to 3e also have recesses 34 fitting the projections 33 of the culture trays like the culture tray 3b. The culture trays 3c to 3e can also be easily stacked properly by putting the projections 33 into the recesses 34. The stacked surrounding walls 32 of the culture trays 3 constitute a sidewall 22 of the culture vessel part 2. The bottom wall 31 of the culture tray 3a on which the culture trays are stacked constitutes the bottom of the culture vessel part 2. Further, recesses may be provided on each surrounding wall 32 of the culture trays 3a to 3e, and protrusions may be provided on the outer surfaces of the bottom walls of the culture trays 3b to 3e and the surfaces facing the culture trays of a cover wall part 41 of the cover plate 4.

The culture vessel part 2 is a substantially rectangular parallelepiped and comprises the bottom wall 31 of the culture tray 3a, the cover wall part 41 that is stacked on the culture tray 3e and faces the bottom wall 31, and an internal space defined by the sidewall 22 connecting the bottom wall 31 and the cover wall part 41. The sidewall 22 has a sidewall part 22d to which the reservoir part 5 is attached, a sidewall part 22b facing the sidewall part 22d, and a sidewall part 22a and a sidewall part 22c which face each other and connect the sidewall part 22b and the sidewall part 22d, as described below with reference to Fig. 2. The internal space of the culture vessel part 2 is partitioned by the culture trays 3. Individual culture trays have the partitioned internal space in the culture vessel part 2 as the internal space of the culture tray. In the case of the culture tray 3a on which the culture tray 3b stacks, the internal space of the culture tray is a space defined by the bottom wall and the surrounding wall of the culture tray 3a and the bottom wall of the culture tray 3b. Each of the culture trays 3b to 3d also has an internal space defined by its bottom wall 31 and surrounding wall 32, and the bottom wall 31 of the individual culture trays 3c to 3e that are stacked. The culture tray 3e has an internal space defined by its bottom wall 31 and surrounding wall 32, and a first cover wall part 41a of the stacked cover plate 4. The internal space of the culture tray 3 can be used as a culture vessel. The internal space of the culture tray 3 of Embodiment 1 is defined by the bottom wall 31 of the culture tray 3 and the surrounding wall 32 surrounding the bottom wall 31.

The cover plate 4 is stacked on the culture tray 3e along the stacking direction (D1) where the culture trays 3a to 3e are stacked. The cover plate 4 comprises a cover wall part 41 that is substantially flat. The cover wall part 41 comprises a first cover wall part 41a stacked on the culture tray 3e and a second cover wall part 41b attached to the reservoir part 5. By attaching the reservoir part 5 to the second cover wall part 41b integrally molded with the first cover wall part 41a of the cover plate 4, the assembled multilayer culture vessel 1 can increase its strength. The cover plate stacked on the at least culture trays 3a to 3e constitutes the upper part of the culture vessel part 2.

The first cover wall part 41a is substantially flat and comprises a cover surface whose shape is substantially the same as the tray surface of the culture tray 3 that is substantially rectangular. The first cover wall part 41a has recesses 44a, which fit the penetrations 33 on the surrounding wall 32 of the culture tray 3e, on the cover surface facing the culture tray 3e. The cover plate 4 can be easily stacked on the culture tray 3e properly by putting the projections 33 of the culture tray 3e into the recesses 44a of the cover wall part 41a.

The second cover wall part 41b is substantially flat and comprises a cover surface whose shape is substantially the same as an inner surface of a surrounding wall part 52a of a reservoir container 50 that is substantially rectangular, described below. The second cover wall part 41b has recesses 44b, which fit projections 53 on four surrounding wall parts 52b, 52c, 52d, and 52e of the reservoir container 50 described below, on the cover surface facing the reservoir part 5. The cover plate 4 can be easily attached to the reservoir part 5 properly by putting the protrusions 53 of the reservoir container 50 into the recesses 44b of the second cover wall part 41b.

The cover plate 4 has a ridge 43 formed around an edge. The ridge 43 facilitates stacking multilayer culture vessels 1. When stacking a multilayer culture vessel 1 on another multilayer culture vessel 1 whose cover plate 4 has a ridge 43, a bottom wall 31 of the culture tray 3 constituting the bottom of the multilayer culture vessel 1 that stacks may have a positioning protrusion fitting the ridge 43. The fitting of the ridge 43 to the positioning protrusion allows preventing the stacked multilayer culture vessels 1 from being out of position.

The reservoir part 5 comprises a reservoir container 50 that is substantially rectangular parallelepiped and has an internal space. The reservoir part 50 is attached to the culture trays 3a to 3e and the cover plate 4. The internal space of the reservoir container 50 is defined by a surrounding wall 52 and the second cover wall part 41b of the cover plate 41. The surrounding wall 52 comprises the surrounding wall part 52a whose inner surface is substantially rectangular and parallel to the tray surface (inner surface) of the bottom wall 31 of the culture tray 3a and constitutes a part of the bottom of the culture vessel part 2. The surrounding wall 52 further comprises four surrounding wall parts 52b, 52c, 52d, and 52e, which extend substantially vertically upward from each side of the surrounding wall part 52a and have predetermined lengths.

The surrounding wall parts 52b to 52e have protrusions 53 that facilitate properly attaching the reservoir part 5 to the cover plate 4. As described above, the reservoir part 5 can be easily attached properly to the cover plate 4 by fitting the propagations 53 of the reservoir container 50 to the recess of the second cover wall part 41b. Further, recesses may be provided on the reservoir part 5, and protrusions 53 may be provided on the second cover wall part 41b.

The surrounding wall part 52e of the reservoir container 50 has a socket 54 that facilitates properly attaching the reservoir part 5 to the culture trays 3. Peripheral wall parts of the surrounding walls 32 of the culture trays 3 facing the reservoir container 50 have locking protrusions 35 fitting the socket 54. The reservoir part 5 can be easily attached to the culture trays 3 properly by fitting the socket 54 of the reservoir container 50 to the locking projections 35 of the culture trays 3.

The surrounding wall part 52e of the reservoir container 50 abuts on the abutting portions 36 provided on the peripheral wall parts of the surrounding walls 32 of the culture trays 3. Adhesion of the abutting portions 36 by, for example, heat bond, melt bond, ultrasonic fusion, or adhesive, can increase the strength of the multilayer culture vessel 1 after being assembled.

The screw port 55 is combined with a corresponding screw lid 56 to constitute an openable port. The screw port 55 is provided on the surrounding wall part 52b of the reservoir container 50 such that an angle formed between the port axis D2 and the stacking direction D1 is about 90 degrees. The surrounding wall part 52b with the screw port 55 is also referred to a first surrounding wall part 52b, and the surrounding wall part 52d facing the surrounding wall part 52b is also referred to a second surrounding wall part 52d. The screw port 55 is provided on the reservoir part 5 such that the port axis D2 is substantially parallel to a surface where the reservoir container 50 is opposed to the culture trays 3.

Openings are provided on the surrounding walls 32 of culture trays 3, the openings communicating the internal space of the reservoir part 5 with the internal spaces of the culture trays 3. The opening of the culture tray 3a is a slit-shaped space formed by the bottom wall 31 of the culture tray 3b stacked on the culture tray 3a and a cutout part 38 provided on the locking protrusion 35 of the culture tray 3a distally from the bottom wall 31. The openings of the culture trays 3b to 3d are slit-shaped spaces formed by the bottom walls 31 of the stacked culture trays 3c to 3e and cutout parts 38 provided on the locking protrusions 35 of individual culture trays 3b to 3d. In addition, the opening of the culture tray 3e is a slit-shaped space formed by the first cover wall part 41a of the cover plate 4 stacked.

The surrounding wall part 52b with the screw port 55 abuts on the sidewall 22d of the culture vessel part 2 at the first abutting portion P1, described below with reference to Fig. 2d. The surrounding wall part 52d facing the surrounding wall part 52b abuts on the sidewall part 22d of the culture vessel part 2 at the second abutting portion P2. The culture vessel part 2 is liquid-tightly bonded to the reservoir part 5 at the first abutting portion P1 and/or the second abutting portion P2 by, for example, heat bond, melt bond, ultrasonic fusion, or adhesive.

An opening provided on the surrounding wall 32 of the culture tray 3 is described in detail with reference to Fig. 2. Fig. 2a shows a multilayer culture vessel 1 according to Embodiment 1, assembled with respective parts shown in Fig. 1. In the multilayer culture vessel 1, a sidewall 22 is constituted by the surrounding walls 32 of five stacked culture trays 3 and a reservoir part 5 is attached to one sidewall part 22d. A culture vessel part 2 has a first sidewall and a second sidewall, both of which are opposed to each other along a first direction where the first surrounding wall part 52b is opposed to the second surrounding wall part 52d. In Fig. 2a, the first sidewall is the sidewall part 22a, and the second sidewall is the sidewall part 22c. The port comprising the screw port 50 and the screw lid 56 is located between a position of the sidewall part 22a in the first direction and a position of the sidewall part 22c in the first direction. That is, the port is below the plane including the sidewall part 22c and is above the plane including the sidewall part 22a, when the sidewall part 22a is placed downward in Fig. 2a. The reservoir part 5 comprises a surrounding wall part 52c and a surrounding wall part 52e, both of which are opposed to each other along a second direction orthogonal to both the first direction and the stacking direction D1 where the at least two trays stack, and the port is located between a position of the surrounding wall part 52c in the second direction and a position of the surrounding wall part 52e in the second direction. That is, the port is the left side from the plane including the sidewall part 22c and is the right side from the plane including the sidewall 52e, when the sidewall part 22b is placed leftward in Fig. 2a. When the port is provided at the position described above, the port is inside the outer peripheral surfaces of the multilayer culture vessel 2 and the reservoir part 5, so that the vessel can be transported efficiently. In addition, the vessel can reduce the risk of the worker's hands or work equipment touching the port during culture workings, and the operability is good.

Fig. 2b shows a cross-sectional view taken along with the A-A line in Fig. 2a. As shown in Fig. 2b, the internal space of the culture vessel part 2 of the multilayer culture vessel 1 is partitioned into five spaces by the five culture trays 3a to 3e. Each of the five internal spaces is adjacent to the internal space of the reservoir container 50 of the reservoir part 5 via the sidewall part 22d, which comprises the locking protrusion 35 of the culture tray 3, of the culture vessel part 2. In the A-A cross-section, the internal space of the reservoir container 50 is surrounded by the second cover wall part 41b, the surrounding wall parts 52a to 52c, and the sidewall 22d.

Fig. 2c is an enlarged view of the part surrounded by the broken lines in Fig. 2b. It is described that the internal spaces partitioned by the culture trays 3 are communicated with the internal space of the reservoir container 50 through the openings 39 formed on the sidewall part 22d of the culture vessel part 2 with reference to Fig. 2c. As described above, the opening 39 formed on the sidewall part 22d is a space formed between the first cover wall part 41a of the cover plate 4 stacked on the culture tray 3 or the bottom wall 31 of the stacked culture tray 3 and a cutout part 38 formed on the locking protrusion 35, the cutout part being distal to the bottom wall 31. As shown in Fig. 2c, each of the internal spaces partitioned by the culture trays 3 is communicated with the internal space of the reservoir container 50 through the opening 39 formed for the individual culture trays 3.

Fig. 2d shows a cross-sectional view taken along the D-D line in Fig. 2a, and the cross-section taken along the D-D line corresponds to a surface where the sidewall part 22d of the culture vessel part 2 of the multilayer culture vessel 1 faces the reservoir container 50. As described above, the openings 39 formed on the sidewall part 22d of the culture vessel part are formed between the first abutting portion P1 that the surrounding wall part 52b abuts on the sidewall part 22d of the culture vessel part and the second abutting portion P2 that the surrounding wall part 52d abuts. In Fig. 2d, the openings 39 formed on the sidewall part 22d extend toward the first abutting portion P1 from the second abutting portion P2 with a length of about one-third of the linear distance between the first abutting portion and the second abutting portion. In the multilayer culture vessel 1 of Embodiment 1, the openings 39 formed on the sidewall part 22d have a length corresponding to that of the locking protrusion fitted to the internal wall parts 54a and 54b of the socket on the surrounding wall part 52e (a cross-section thereof is shown). The length of the openings 39 to be less than two-thirds of the linear distance between P1 and P2 is preferably set so that the mechanical strength of the multilayer culture vessel 1 including the culture vessel part 2 and the reservoir container 50 can be stronger.

The size of the multilayer culture vessel 1 of Embodiment 1 is described with reference to Fig. 2. When the short side of the multilayer culture vessel 1 has a length L1 of 100 to 150 mm in Fig. 2a, the long side of the multilayer culture vessel 1 has, for example, a length L2 of 200 to 300 mm, and the long side of the culture vessel part 2 has, for example, a length L8 of 180 to 210 mm. As shown in Fig. 2a, the reservoir container 50 is attached to the sidewall part 22d of the multilayer culture vessel 1 at an upper position from the horizontal position of the sidewall part 22a. The distance L3 of the upper position is, for example, 8 to 13 mm when the short side of the multilayer culture vessel 1 has a length L1 of 100 to 150 mm. In the first posture shown in Fig. 3b, lifting the sidewall part 22b allows to easily tilt the reservoir part 5 of the multilayer culture vessel 1 diagonally downward because of being attached at the upper position, so that users can easily inject or collect a fluid sample through the port. When multilayer culture vessels with relatively large size are used in the limited workspace in clean benches, there is a high possibility that worker's fingers will come into contact with the port, resulting in an increase in the risk of contaminating the inside of the multilayer culture vessel. In addition, when collecting a fluid sample from a relatively large multilayer culture vessel with a relatively short instrument such as a pipette, it is hard to collect the sample from the reservoir part, so that sample waste is likely to occur. The length L1 of the multilayer culture vessel is preferably not more than 140 mm to prevent samples waste and decrease work efficiency.

In Fig. 2b, the internal spaces partitioned by the cultures trays 3 have, for example, a height L4 of 5 to 10 mm when the short side of the multilayer culture vessel 1 has a length L1 of 100 to 150 mm. The multilayer culture vessel 1 has, for example, a height L5 of 45 to 55 mm when the short side of the multilayer culture vessel 1 has a length L1 of 100 to 150 mm. In the multilayer culture vessel 1 of Embodiment 1, the bottom walls 31 of the culture trays 3 and the cover wall part 41 of the cover plate 4 have, for example, a thickness of 1.5 to 2.5 mm, respectively.

In Fig. 2d, among the contact portions where the reservoir container 50 abuts on the culture vessel part 2, the contact portion at the upper end that the surrounding wall portion 52b provided with the screw port 55 abuts is referred to a first abutting portion P1 ant the contact portion at the lower end is referred to a second contact portion P2. When the distance between the abutting portions is 75 to 90 mm, the openings 39 formed on the sidewall part 22d extend toward the first abutting portion P1 from the second abutting portion P2 with a length of, for example, 25 to 35 mm (L6).

When the openings 39 formed on the sidewall parts 22d have a longitudinal length L6 of 25 to 35 mm, the openings 39 have, for example, a width L7 of 1 to 2 mm. The openings 39 extend toward the first abutting portion P1 through the second abutting portion P2 from the sidewall part 22a of the culture vessel part 2 with a distance L10. The distance L10 is from the sidewall part 22a of the culture vessel part 2 to the opening edge of the short side, which is located near the first abutting portion P1, among the opening edges of the openings 39. In a multilayer culture vessel according to Embodiment 2 described below, the reservoir container is attached to the culture vessel part at the same level as the first sidewall part 22a'. Accordingly, the distance L6 is the same value as the distance L10. In the multilayer culture vessel according to Embodiment 1, when the openings 39 have a length L6 of 25 to 35 mm in the longitudinal direction, the distance L10 is, for example, 33 to 48 mm.

In Fig. 2c, the width L7 of the opening 39 is a distance between a lower edge 39a and an upper edge 39b of the opening. The upper edge 39b of the opening corresponds to the outer surface of the cover wall part 41 of the stacked cover plate or the outer surface of the bottom wall 31 of the culture trays. When the height L4 of the internal spaces partitioned by the culture trays 3 is 5 to 10 mm, the distance L9 from the bottom wall 31 of the culture trays to the lower edge 39a of the opening is, for example, 3 to 9 mm.

The way to use the multilayer culture vessel 1 after injecting a fluid sample (for example, culture medium) is described with reference to Fig. 3. Fig. 3a shows a cross-sectional view taken along the E-E line in Fig. 2 and shows the multilayer culture vessel 1 placed in the first posture after the fluid sample is injected. In the first posture of the multilayer culture vessel 1, the multilayer culture vessel 1 is placed so that the sidewall part 22a of the culture vessel part 2 faces downward and the port 55 provided in the reservoir part 5 attached to the culture vessel part 2 faces upward. The multilayer culture vessel 1 placed in the first posture allows users to easily inject a fluid sample to or collect the fluid sample from the internal space defined by the culture trays of the culture vessel part 2 through the port 55.

As shown in Fig. 3a, the liquid level of the fluid sample in the reservoir container 50 in the first posture is the same for each of the openings 39 formed in the sidewall part 22d. As described above, each of the internal spaces of the culture vessel part 2 partitioned by the culture trays 3 communicates with the internal space of the reservoir container 50 through the openings 39 formed in sidewall part 22d. When a fluid sample is injected into the internal space of the reservoir container 50 through the port 55, the injected fluid sample flows into each of the internal spaces in the culture vessel part 2 through each of the openings 39 formed in the sidewall part 22d.

Fig. 3b shows the cross-section of the multilayer culture vessel 1 in the first posture, taken along with the B-B line in Fig. 2b. As shown in Fig. 3b, the fluid sample flowed into the respective internal spaces of the culture vessel part 2 through the openings formed in the sidewall part 22d of the culture vessel part 2 has the same liquid level as the fluid sample in the reservoir container 50. According to the multilayer culture vessel 1, an injected fluid sample evenly flows into the respective internal spaces partitioned by the culture trays 3 in the culture vessel part 2 just by performing injection work, as seen from Figs. 3a and 3b.

Fig. 3c shows the multilayer culture vessel 1 placed in the second posture so that the sidewall part 22b of the culture vessel part 2 faces downward. The second posture is a posture after the multilayer culture vessel 1 in the first posture is rotated 90 degrees in the direction along with the arrow R in Fig. 3b. During the transition from the first posture to the second posture, the fluid sample held in the internal space of the reservoir container 50 flows into the internal spaces partitioned by the culture trays 3 of the culture vessel part 2 through the openings 39 formed in the sidewall part 22d. Accordingly, all the fluid sample injected into the reservoir container 50 are basically distributed to the internal spaces partitioned by the culture trays of the culture vessel part 2, so that the waste of fluid samples can be eliminated.

Fig. 3d shows the multilayer culture vessel 1 placed in the third posture so that the bottom wall 31 of the culture tray 3a faces downward by tilting it 90 degrees toward the back centering on the sidewall part 22b from the second posture shown in Fig. 3c. As shown in Fig. 3a to 3d, it is easy to realize the state in which the fluid sample is evenly distributed to the individual culture trays 3a, 3b, 3c, 3d, and 3e, constituting the culture vessel part 2, by changing the postures of the multilayer culture vessel 1 from the first posture through the second posture to the third posture. The liquid level of the fluid sample distributed to the individual culture trays is lower than the cutout parts forming the openings 39 of the individual culture trays. As mentioned above, according to the multilayer culture vessel 1 in the first posture, the fluid sample is evenly distributed to the internal spaces partitioned by the culture trays 3 of the culture vessel part 2 just by injecting the fluid sample, so that culture conditions can be easily uniform. This can suppress the waste of reagents caused by the non-uniformity of the state of cultured cells.

Contamination in cell culture using culture vessels generally more likely occurs near the port, which provides communication between the internal space of a culture vessel and the external space. For this reason, cell culture operations are usually performed so that fluid samples contacted with the port do not enter the culture vessel. As seen from Fig. 3, the multilayer culture vessel 1 according to Embodiment 1 basically allows the fluid sample injected from the port 55 to avoid contact with the port until the injected liquid sample is distributed to the individual internal structures partitioned by the culture trays 3 of the culture vessel part 2. The multilayer culture vessel 1 according to Embodiment 1 is also advantageous from the viewpoint of contamination risk.

A sidewall part constitution is not limited to Embodiment 1 where the sidewall part 22 of the culture vessel part 2 is constituted of the surrounding walls 32 of the stacked at least two culture trays 3. A sidewall of the culture vessel part may be, for example, a sidewall of a container constituting the culture vessel part. In the above example, at least two culture trays of the multilayer culture vessel may be stacked such that outer peripheral surfaces of bottom walls or outer surface of surrounding walls of the culture trays are bonded to the internal surface of the sidewall of the container of the culture vessel part. Accordingly, Embodiment 1 of the present invention provides a variation described below.

[Another embodiment]

A multilayer culture vessel, comprising a culture vessel part including a storage container and a reservoir part attached to the culture vessel part, wherein the storage container comprises at least two culture trays that are stacked; the reservoir part comprises a surrounding wall defining an internal space and a port communicating with the internal space; the surrounding wall comprises a first surrounding wall part provided with the port and a second surrounding wall part facing the first surrounding wall part; the culture vessel part has openings communicating individual culture trays of the at least two culture trays with the internal space of the reservoir part; and the openings of the culture vessel part extend toward a first abutting portion that the first surrounding wall part abuts on the culture vessel part from a second abutting portion that the second surrounding wall part abuts on the culture vessel part.

The stacking manner is not limited to Embodiment 1 in which at least two culture trays are stacked such that the surrounding wall 32 of one culture tray 3 contacts with the bottom wall of another culture tray 3. For example, in Another embodiment of Embodiment 1, outer surfaces of the bottom walls or outer surfaces of the surrounding walls of the culture trays are bonded to the internal surface of the sidewall of the storage container that constitutes the culture vessel part. Thereby, the culture trays' bottom walls or tray surfaces are stacked such that the surrounding wall of one culture tray does not contact with the bottom wall of another culture tray.

The stacking order is not limited to that of Embodiment 1 in which the multilayer culture vessel 1 is assembled by stacking the at least two culture trays 3 on the culture tray 3a in the order of the culture trays 3b, 3c, 3d, and 3e. For example, when outer surfaces of the surrounding walls of the culture trays are bonded to the internal surface of the sidewall of the storage container constituting the culture vessel part in Another embodiment of Embodiment 1, the order in which the culture trays stack, that is, the order in which the outer surfaces of the bottom walls or the outer surfaces of the surrounding walls of the culture trays are bonded to the internal surface of the sidewall of the container constituting the culture vessel part is not particularly limited.

The culture tray structure is not limited to that of Example 1 in which the culture tray 3 comprises a surrounding wall 32 composed of peripheral wall parts extending from the outer periphery of the bottom wall 31 and are integrally molded with the bottom wall 31. For example, in Another embodiment of Embodiment 1, the culture tray comprises a bottom wall, whose outer peripheral surface is bonded to wall parts of the storage container of the culture vessel part, and the wall parts as a surrounding wall of the culture tray. The surrounding wall of the culture tray is composed of, for example, peripheral wall parts extending from the bottom wall, wall parts attached to the bottom wall, or peripheral wall parts extending from the bottom wall and wall parts attached to the bottom wall.

The culture tray structure is not limited to that of Embodiment 1 in which the surrounding wall 32 of the culture tray 3 comprises peripheral wall parts extending substantially vertically upward from the outer peripheral of the bottom wall. The surrounding walls of the culture trays may comprise peripheral wall parts extending diagonally upward from the outer periphery of the bottom wall.

The culture tray structure is not limited to that of Embodiment 1 in which the culture tray 3 comprises the bottom wall 31 whose surface is substantially flat and rectangular. The tray surface may be triangular, square, pentagonal, hexagonal, oval, and circular. The tray surface of the bottom wall of a culture tray constitutes, for example, the bottom of the culture vessel when placed in a posture suitable for culturing in a multilayer culture vessel.

The number of trays is not limited to that of Embodiment 1 in which at least culture trays 3 that are stacked comprise five culture trays 3a to 3e. The number of the at least culture trays stacked may be appropriately set by those skilled in the art according to the purpose and may be, for example, two, three, four, five, six, or more.

The culture tray structure is not limited to that of Embodiment 1 in which the at least two culture trays 3 stacked have substantially the same shape as each other. For example, the culture tray constituting the bottom of the multilayer culture vessel, when placed in a posture suitable for culturing in a multilayer culture vessel, may have a different shape than the other culture trays stacked on the tray. For example, the culture tray constituting the bottom surface of the culture vessel part may be provided with protrusions to prevent the bottom of the multilayer culture vessel from directly touching a culture apparatus when the vessel is placed in the culture apparatus.

The cover plate structure is not limited to that of Embodiment 1 in which the cover plate 4 comprises a substantially flat cover wall part 41. The cover plate structure is not limited to that of Embodiment 1 in which the cover plate 4 comprises the first cover wall part 41a stacked on the culture tray 3e and the second cover wall part 41b attached to the reservoir part 5. The first cover wall part may constitute the cover plate stacked on culture trays.

The cover plate is not limited to that of Embodiment 1 in which the cover plate 4 is stacked on the surrounding wall 32 of the at least two culture trays 3. For example, when the culture vessel part comprises a storage container like Another embodiment of Embodiment 1, the upper part of the storage container serves as a cover plate.

The reservoir part structure is not limited to that of Embodiment 1, in which the reservoir part 5 is attached to the sidewall part 22d of the culture vessel part and the second cover wall part 41b of the cover plate 4. For example, the reservoir part may be attached to only the sidewall part 22d of the culture vessel part when the reservoir part, instead of cover plate 4, has a surrounding wall corresponding to the second cover wall part 41b. In another example, when the bottom wall 31 of the culture tray 3, instead of the reservoir part, is integrally formed with an additional bottom wall corresponding to the surrounding wall part 52a, the reservoir part may be attached to the sidewall part 22d of the culture vessel part, the second cover wall part 41b of the cover plate 4, and the additional bottom wall of the culture tray 3. In this example, the assembled multilayer culture vessel 1 has a relatively high mechanical strength and is preferable.

The reservoir part structure is not limited to that of Embodiment 1 in which the reservoir part 5 comprises a reservoir container 50 that is a roughly rectangular parallelepiped. The shape of the reservoir part 5 may be, for example, regular hexahedron, truncated cone, polygonal pillar such as a pentagonal pillar.

The assembling manner is not limited to Embodiment 1 in which the culture vessel part 2 is attached to the reservoir part 5 by fitting the locking protrusions 35 formed on the surrounding walls 32 of the culture trays 3 to the socket 54 formed on the reservoir container 50 and bonding the surrounding wall part 52e of the reservoir container 50 to the abutting portion 36 of the surrounding wall 32 of the culture trays. The attachment may be, for example, either fitting the locking protrusion to the socket or bonding at the abutting portion. In another example, the culture vessel part 2 and the reservoir part 5 may be integrally molded.

In Embodiment 1, the first surrounding wall part 52b, which is provided with port 55, of the surrounding wall 52 constituting the reservoir container 50 is substantially parallel to the second surrounding wall part 52d facing the first surrounding wall part 52b, that is, the angle formed between the first surrounding wall part 52b and the second surrounding wall part 52d faced to each other is about 0 degrees, but the angle is not limited to about 0 degrees. The angle formed between the first surrounding wall part provided with the port and the second surrounding wall part facing thereto may be, but not limited to, not less than 0 degrees to not more than 50 degrees, for example, not less than 0 degrees to not more than 45 degrees, not less than 0 degrees to not more than 40 degrees, not less than 0 degrees to not more than 35 degrees, not less than 0 degrees to not more than 30 degrees, not less than 0 degrees to not more than 20 degrees, not less than 0 degrees to not more than 10 degrees, or not less than 0 degrees not more than 5 degrees. The angle formed between the first surrounding wall part provided with the port and the second surrounding wall part facing thereto may be, for example, 0 degrees, 10 degrees, 20 degrees, 30 degrees, 35 degrees, 40 degrees, 45 degrees, or 50 degrees.

The port shape is not limited to that of Embodiment 1 in which the shape of the port 55 is cylindrical convex pipe. The shape of the port is, for example, not particularly limited as long as fluid samples can be injected and collected with a dispensing device such as pipette. The shape of the port may be, for example, convex pipe, concave pipe, or hole. The port has, for example, an opening surface of circular, elliptical, or polygonal (e.g., hexagonal).

The port structure is not limited to that of Embodiment 1 in which the port 55 is an openable screw port with the screw lid 56. The port may be, for example, an opening with a hinge cap. The material of the lid used as a port is, for example, metal or plastic such as stainless steel or polyethylene, commonly used in pharmaceutical fields or research fields. The lid may be, for example, a bent cap having filter with pores or hydrophobic membrane capable of exchanging gas. The pores may have, for example, sizes capable of protecting cells in containers from bacterial or virus contamination. The pores are, for example, less than 0.65 micron, 0.4 micron, or 0.22 micron.

The angle formed between the axis D2 of port 55 and the stacking direction D1 is not limited to that of Embodiment 1 in which the angle is about 90 degrees. The angle formed between the port axis D2 and the stacking direction D1 may be, for example, not less than 70 degrees to not more than 90 degrees, not less than 75 degrees to not more than 90 degrees, not less than 80 degrees to not more than 90 degrees, and not less than 85 degrees to not more than 90 degrees. The angle formed between the port axis D2 and the stacking direction D1 may be, for example, 90 degrees, 85 degrees, 80 degrees, 75 degrees, or 70 degrees.

In Embodiment 1, the port 55 is provided on the surrounding wall part 52b of the reservoir container 50 such that the axis D2 is substantially parallel to the surface facing the side wall part 22d of the culture vessel part 2 to the surrounding wall part 52e of the reservoir container 50, that is, the angle formed between the axis D2 of port 55 and the surface facing the sidewall part 22d of the culture vessel part 2 to the surrounding wall part 52e of the reservoir container 50 is about 0 degrees, but the angle is not limited to about 0 degrees. The angle formed between the axis D2 of port and the surface facing the sidewall part of the culture vessel part to the surrounding wall part of the reservoir container may be, but not limited to, not less than 0 degrees to not more than 50 degrees, for example, not less than 0 degrees to not more than 45 degrees, not less than 0 degrees to not more than 40 degrees, not less than 0 degrees to not more than 35 degrees, not less than 0 degrees to not more than 30 degrees, not less than 0 degrees to not more than 20 degrees, not less than 0 degrees to not more than 10 degrees, or not less than 0 degrees to not more than 5 degrees. The angle formed between the first surrounding wall part provided with the port and the second surrounding wall part facing it is, for example, 0 degrees, 10 degrees, 20 degrees, 30 degrees, 35 degrees, 40 degrees, 45 degrees, or 50 degrees.

The surface facing the side wall part 22d of the culture vessel part 2 to the side wall 52e of the reservoir container 50 is, for example, a flat surface including two portions at both ends of the second abutting portion P2 and the nearest portion between the axis D2 of the port 55 and the first abutting portion P1.

The openings are not limited to that of Embodiment 1 in which the openings 39 of the culture vessel part 2 are formed by the cutout parts 38 provided on the surrounding wall 32 of the culture tray 3. The opening of the culture vessel part may be, for example, a hole or aperture provided in a peripheral wall part extending from the bottom wall. In another example, the openings of the culture vessel part may be spaces formed between culture trays stacked such that a surrounding wall of one of the culture trays does not contact with a bottom wall of the other culture tray like Another embodiment of Embodiment 1.

The openings are not limited to that of Embodiment 1 in which the openings 39 of the culture vessel part 2 are formed by stacking the culture trays 3 such that the cutout parts 38 formed on the surrounding walls 32 are aligned with a surface that the sidewall parts 22d of the culture vessel parts 2 are opposed to the surrounding wall part 55e of the reservoir container 50. Openings whose opening surfaces face toward the stacking direction can be formed by stacking culture trays with locking protrusions whose lengths protruding from the surrounding wall are different and shorter as the stacking direction advances.

The openings are not limited to that of Embodiment 1 in which the culture vessel part 2 has one opening 39 per culture tray 3. The culture trays may have two or more openings per culture tray. In the case where a fluid sample is injected through the port 55 into the culture vessel part 2 of the multilayer culture vessel 1 placed in the first posture shown in Fig. 3a and the liquid level of the injected fluid is above the surrounding wall part 52e of the reservoir container 50, the fluid sample does not flow into the culture vessel part 2 due to the air remaining in the internal space of the culture trays of culture vessel part 2. The culture vessel part 2 may have additional openings on the sidewall part 22d, which allow the remaining air to escape so that the fluid sample flows into the culture vessel part 2 even if the fluid level of the fluid sample is above the surrounding wall part 52e of the reservoir container 50. The additional openings are formed above the openings 39 in the multilayer culture vessel 1 placed in the first posture shown in Fig. 3a.

In the case where no additional openings allowing the remaining air to escape are provided in the culture vessel part 2, the surrounding wall part 52e of the reservoir container 50 of the multilayer culture vessel 1 acts as a regulatory for adjusting the amount of a fluid sample flowing into the culture vessel part 2, so that the culture trays hold the fluid sample and the air at a predetermined volume ratio.

The position of the opening is not limited to that of Embodiment 1 in which the openings 39 of the culture vessel part 2 are formed on the surrounding walls 32 of culture trays 3 such that the lower edges 39a of the openings are formed at the position of about four-fifths of the surrounding walls' height. The openings of the culture vessel part 2 may be formed, for example, on the surrounding walls of the culture trays such that the lower edges 39a of the openings are formed at the position of more than half, more than three-fifths, more than two-thirds, more than three-quarters, or more than four-fifths of the surrounding walls' heights.

When the multilayer culture vessel is placed in a posture (third posture) suitable for culturing as shown in Fig. 3d, the distance L9, which is from the tray surface of the bottom wall 31 of each culture tray 3 of the culture vessel part 2 to the lower end 39a of the opening formed on the surrounding wall 32 of said each culture tray 3, limits the upper level of the fluid sample that can be held in the culture tray. For example, in the case where a lower end 39a of the opening of the culture vessel part is formed at half the height of the surrounding wall 32 of the culture tray and a fluid sample introduced into the internal space of the culture tray in an amount that the liquid level exceeds the half-height, a part of the fluid sample overflows from the opening. Accordingly, the space formed by the tray surface of the bottom wall 31 of the culture tray 3 and the distance L9 to the lower end 39a of the opening, among the internal spaces partitioned by the culture trays 3 in the culture vessel parts 2, corresponds to the storage space that can be used as a culture vessel. To prevent a part of the fluid sample from overflowing from the internal spaces of the culture trays to the reservoir container 50 through the openings 39 when the posture of the multilayer culture vessel 1 is changed from the first posture through the second posture to the third posture, an indication for the appropriate amount of fluid sample in the multilayer culture vessel 1 placed in the first posture may be marked on the culture vessel part 2 and/or the reservoir part 5. Such marks may be printed, embossed, or sealed.

A maximum volumetric capacity that the culture vessel part 2 can hold when the multilayer culture vessel 1 is placed in the third posture is calculated, for example, by multiplying the area of the tray surface of the bottom wall 31 of the culture trays (L1 × L8, see Fig. 2a) by the distance L9 from the bottom wall 31 of the culture trays to the lower end 39a of the opening to obtain a volumetric capacity (a maximum capacity that one culture tray 3 can hold) and by multiplying the volumetric capacity by the number of the culture trays 3 constituting the culture vessel part 2 (L1 × L8 × L9 × [the number of culture trays]). The above-mentioned mark indicating an appropriate amount of fluid sample in the first posture may be put, for example, at a position corresponding to a volumetric capacity not more than the maximum volumetric capacity that the culture vessel part 2 can hold. There is a risk that the liquid sample overflows from the culture trays 3 to the reservoir container 50 (50') due to vibration or the like, in a particular case where the liquid sample is held up to the maximum volumetric capacity that can be held in the third posture. Accordingly, the amount of liquid sample to be held or the tray area of culture tray 3 may be appropriately set, preferably such that the liquid volume is less than 50% of the distance L9. The appropriate volume to be held in the culture vessel part may be expressed as L1 × L8 × L9 × 0.5 × [the number of culture trays].

When air bubbles form on the fluid sample, the amount of the collected fluid sample decreases. Further, when air bubbles burst, fluid sample droplets scatter, which may cause contamination. An internal structure of the multilayer culture vessel may facilitate forming air bubbles on the fluid sample. For example, in the case where the posture of the multilayer culture vessel 1 is changed from the third posture to the first posture to collect culture medium held in the multilayer culture vessel, air bubbles are likely to be formed if the liquid level of the fluid sample is higher than the position of the opening edge of the short side, which is located near the first abutting portion P1, of the opening formed on the surrounding wall of each culture tray. The opening edge of the short side, which is located near the first abutting portion P1, of the opening formed on the surrounding wall of each culture tray is preferably positioned higher than the liquid level of the appropriate volume to be held in the culture vessel part 2 in order to suppress the formation of air bubbles, wherein the liquid level corresponds to the appropriate volume when the culture vessel is placed in the first position. In other words, the volumetric capacity obtained by multiplying the area of the bottom surface of the multilayer culture vessel placed in the first posture by the distance to the opening edge located near the first abutting portion P1 is preferably larger than the appropriate volume to be held in the culture vessel part 2. Such a multilayer culture vessel has, for example, the following relationship:
[volumetric capacity obtained by multiplying the area of the bottom surface of the multilayer culture vessel placed in the first posture by the distance to the opening edge located near the first abutting portion P1] ≥ [appropriate volume to be held in the culture vessel part] (1).

The volumetric capacity of the left side in the inequality (1) can be obtained by multiplying the distance L10 (see Fig. 2d), which is from the sidewall part 22a to the opening edge located near the first abutting portion P1, by the area that is obtained by multiplying the length L2 of the long side of the multilayer culture vessel by the height L5 viewed from the sidewall part 22a of the multilayer culture vessel (see Fig. 2b). Accordingly, the volumetric capacity of the left side can be expressed as L10 × L2 × L5. As described above, the volume of the right side can be expressed as L1 × L8 × L9 × [the number of culture trays]. Substituting these relations into the inequality (1) yields the following formula (2):

$$L10 \times L2 \times L5 \geq L1 \times L8 \times L9 \times [\text{the number of culture trays}] \text{ (Formula 2).}$$

As shown in Fig. 2, the height L5 of the multilayer culture vessel is almost equal to the value obtained by multiplying the height L4 of the internal space of the culture tray by the number of the culture trays constituting the multilayer culture vessel. Accordingly, it can be expressed as L5 ≈ L4 × [the number of culture trays]. Substituting these relationships into the inequality (2) yields the following formula (3):

$$L10 \times L2 \times L4 \times [\text{the number of culture trays}] \geq L1 \times L8 \times (L9 \times 0.5) \times [\text{the number of culture trays}] \text{ (Formula 3).}$$

Dividing both sides of the inequality 3 by L1, L2, L4, and [the number of culture trays] yields formula (4):

$$L10/L1 \geq (L9 \times 0.5)/L4 \times L8/L2 \text{ (Formula 4).}$$

[wherein, L10/L1 (see Fig. 2d) is a ratio of the length L10, which is from the sidewall part 22a to the opening edge located near the first abutting portion P1, to the length L1 of the short side of the multilayer culture vessel; L9/L4 (see Fig. 2c) is a ratio of the distance L9, which is a distance to the opening's lower end 39a limiting the upper level of the fluid sample to be held in the culture tray, to the height L4 of the internal space used as the culture vessel; and L8/L2 (see Fig. 2a) is a ratio of the length L8 of the long side of the culture vessel part 2 to the length L2 of the long side of the multilayer culture vessel 1.

The openings are not limited to that of Embodiment 1 in which the openings 39 of the culture vessel part 2 are formed on the surrounding walls 32 of the culture trays 3. For example, when the culture vessel part is constituted of a storage container like Another embodiment of Embodiment 1, openings of the culture vessel part may be formed on the sidewall of the storage container. In a way, openings on the area where the reservoir part is attached to the culture vessel part are considered the openings of the culture vessel part, even if the openings are on the surrounding wall of the reservoir part in assembling the multilayer culture vessel.

The shape of the openings is not limited to that of Embodiment 1 in which the openings 39 of the culture trays 3 are slit-shaped spaces. The openings of the culture trays may be, for example, triangle or ellipse shape. The openings may be, for example, multiple spaces formed intermittently.

[Embodiment 2]

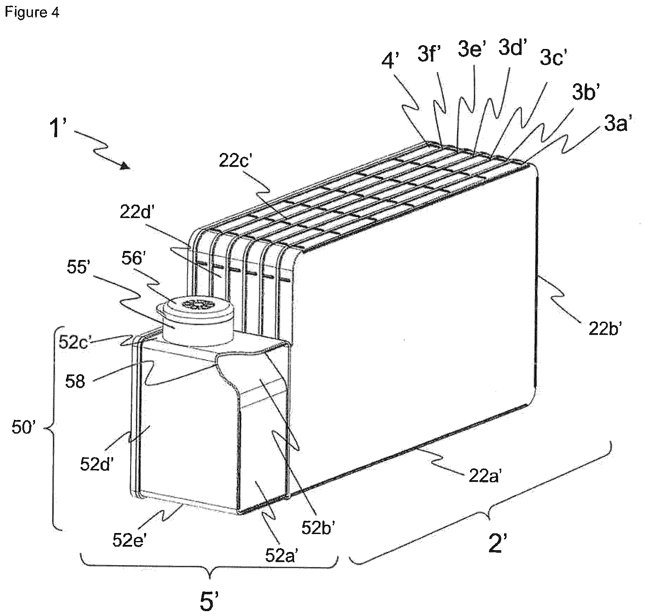

Fig. 4 shows a perspective view of a multilayer culture vessel according to another embodiment of the present invention ("Embodiment 2"). Embodiment 2 is described below with reference to Fig. 4. The differences from Embodiment 1 are mainly described, and the descriptions of similar features are omitted. The multilayer culture vessel 1 of Embodiment 1 comprises five culture trays, while the multilayer culture vessel 1' of Embodiment 2 comprises six culture trays 3a' to 3f.

The port provided in the reservoir part 5 of Embodiment 1 is a screw port 55 with an openable screw lid 56, while the port provided in a reservoir part 5' of Embodiment 2 is an openable cap type port 55' with a hinge cap 56'. The hinge cap 56' is equipped with a gas exchange part having an opening, which communicates the internal space of the reservoir part 5' with the external space and is covered with filter or hydrophobic membrane having pores. For example, the hinge cap 56' of Embodiment 2 shown in Fig. 4 has eight gas exchange parts. The eight gas exchange parts are spaced apart from each other around the center of the hinge cap 56' viewed from its thickness direction, and ribs radiate from the center between adjacent gas exchange parts. The gas exchange parts may be provided on the screw lid. A lid or cap may have one or more gas exchange parts. For example, the lid or cap has three gas exchange parts, and the gas exchange parts are spaced around the center of the lid or cap viewed from its thickness direction, preferably at equal angles.

In the multilayer culture vessel 1 according to Embodiment 1, the surrounding wall part 52d of the reservoir container 50 of the reservoir part 5 is attached to the surrounding sidewall part 22d of the culture vessel part 2 at an upper position from the horizontal position of the sidewall part 22a. In the multilayer culture vessel 1' according to Embodiment 2, the surrounding wall part 52e' of the reservoir container 50' of the reservoir part 5' is attached to the sidewall part 22d' of the culture vessel part 2' at the same level as the sidewall part 22a'. This improves stability when the multilayer culture vessel 1' is placed in the first posture. In addition, when a fluid sample is collected in the first posture, openings of the culture tray 3', which extend from the abutting portion where the surrounding wall part 52e' of the reservoir container 50' abuts on the sidewall part 22d' of the culture tray 3', allow reducing the amount of fluid sample left behind.

The multilayer culture vessel 1' according to Embodiment 2 has a drawer part 58 on the surrounding wall part 52c' and the surrounding wall part 52d' of the reservoir container 50'. The drawer part 58 is convenient for users to hook their fingers and pull the multilayer culture vessel 1' out when delivering the multilayer culture vessel 1' from a culture apparatus after the cell culturing in the culture apparatus. The reservoir container 50' has a surrounding wall part 52b' that is inclined from the surrounding wall part 52a' to form space as the drawer part 58 that fingers can hook.

The descriptions for members such as the culture tray, the cover plate, the reservoir container, and the openings of the culture trays in the multilayer culture vessel according to Embodiment 1 apply to the corresponding members in the multilayer culture vessel according to Embodiment 2.

Another aspect of the present invention provides a method for producing a pharmaceutical composition, the method comprising: culturing cells with a multilayer culture vessel according to an embodiment of the present invention; collecting from the multilayer culture vessel cultured cells or a culture fluid containing a component secreted from the cultured cells; and producing the pharmaceutical composition comprising the collected cultured cells, the collected secreted component, or a component isolated and purified from the collected cultured cells.

Reference Signs

    1 and 1' multilayer culture vessel
    2 and 2' culture vessel part
    3, 3', 3a to 3e, and 3a' to 3f' culture tray
    4 and 4' cover plate
    5 and 5' reservoir part
    22, 22a to 22d, and 22a' to 22d' sidewall
    31 bottom wall
    32 surrounding wall
    33 protrusion
    34 recess
    35 locking protrusion
    36 abutting portion

38 cutout part
39 opening
39a and 39b upper edge and lower edge of an opening
41 cover wall part
41a and 41b first and second cover wall part
43 ridge
44 recess
44b recess
50 and 50' reservoir container
52, 52a to 52e, and 52a' to 52e' surrounding wall part
53 protrusion
54 socket
55 screw port
55' cap type port
56 screw lid
56' hinge cap
58 drawer part
D1 stacking direction
D2 port axis

**Claims**

1. A multilayer culture vessel, comprising a culture vessel part and a reservoir part attached to the culture vessel part, wherein

   the culture vessel part comprises at least two culture trays that are stacked;
   the reservoir part comprises a surrounding wall defining an internal space and a port communicating with the internal space;
   the surrounding wall comprises a first surrounding wall part provided with the port and a second surrounding wall part facing the first surrounding wall part;
   the culture vessel part has openings communicating individual culture trays of the at least two culture trays with the internal space of the reservoir part; and
   the openings of the culture vessel part extend toward a first abutting portion that the first surrounding wall part abuts on the culture vessel part from a second abutting portion that the second surrounding wall part abuts on the culture vessel part.

2. The multilayer culture vessel according to claim 1, wherein the port is provided in the reservoir part such that an angle formed between an axis of the port and a stacking direction that the at least two trays stack is from not less than 70 degrees to not more than 90 degrees.

3. The multilayer culture vessel according to claim 1 or 2, wherein the port is provided in the reservoir part such that an angle formed between an axis of the port and a surface that the reservoir part is opposed to the at least two culture trays is from not less than zero degrees to not more than 50 degrees.

4. The multilayer culture vessel according to any one of claims 1 to 3, wherein the openings of the culture vessel part extend toward the first abutting portion from the second abutting portion with a length of not more than two-thirds of the linear distance between the first abutting portion and the second abutting portion.

5. The multilayer culture vessel according to any one of claims 1 to 4, wherein

   the culture tray comprises a bottom wall and a surrounding wall surrounding the bottom wall;
   the surrounding walls of the stacked at least two culture trays constitute a sidewall of the culture vessel part; and
   the openings of the culture vessel part are formed in the surrounding walls of the individual culture trays constituting the sidewall, distally from the bottom wall of the culture tray.

6. The multilayer culture vessel according to claim 5, wherein

the culture vessel part comprises a cover plate including a cover wall part, the cover plate being stacked on the at least two culture trays;

each culture tray of the at least two culture trays comprises a bottom wall and a surrounding wall surrounding the bottom wall; and

the opening formed in the surrounding wall of each of the culture trays is a space formed between a cutout part formed in the surrounding wall of each of the culture trays and the bottom wall of the culture tray stacked on said each of the culture trays or a space formed between the cutout part and the cover wall part of the cover plate.

7.  The multilayer culture vessel according to any one of claims 1 to 6, wherein

the culture vessel part comprises a first sidewall and a second sidewall, both of which are opposed to each other along a first direction that the first surrounding wall part is opposed to the second surrounding wall part; and

the port is located between a position of the first sidewall in the first direction and a position of the second sidewall in the first direction.

8.  The multilayer culture vessel according to claim 7, wherein a distance between the first sidewall and the second sidewall in the first direction is not more than 140 mm.

9.  The multilayer culture vessel according to any one of claims 1 to 8, wherein

the reservoir part comprises a third surrounding wall part and a fourth surrounding wall part, both of which are opposed to each other along a second direction orthogonal to both the first direction and the stacking direction that the at least two trays stack; and

the port is located between a position of the third surrounding wall part in the second direction and a position of the fourth surrounding wall part in the second direction.

10. A multilayer culture vessel, comprising a culture vessel part and a reservoir part attached to the culture vessel part, wherein

the culture vessel part comprises at least two culture trays that are stacked; the reservoir part has an internal space and comprises a port communicating with the internal space;

the culture vessel part has openings communicating individual culture trays of the at least two culture trays with the internal space of the reservoir part; and

the port is provided in the reservoir part such that an angle formed between an axis of the port and a stacking direction that the at least two trays stack is from not less than 70 degrees to not more than 90 degrees and an angle formed between the axis of the port and a surface that the reservoir part is opposed to the at least two culture trays is from not less than zero degrees to not more than 50 degrees.

11. The multilayer culture vessel according to claim 10, wherein

the reservoir part comprises a surrounding wall defining the internal space, the surrounding wall including the port; the surrounding wall comprises a first surrounding wall part provided with the port and a second surrounding wall part facing the first surrounding wall part; and

the openings of the culture vessel part extend toward a first abutting portion that the first surrounding wall part abuts on the culture vessel part from a second abutting portion that the second surrounding wall part abuts on the culture vessel part.

12. A method for producing a pharmaceutical composition, the method comprising culturing cells with the multilayer culture vessel according to any one of claims 1 to 11;

collecting from the multilayer culture vessel cultured cells or a culture fluid containing a component secreted from the cultured cells; and

producing the pharmaceutical composition comprising the collected cultured cells, the collected secreted component, or a component isolated and purified from the collected cultured cells.

Figure 1

Figure 2

Figure 3

**(a)**

**(b)**

**(c)**

**(d)**

Figure 4

| | INTERNATIONAL SEARCH REPORT | | International application No. |
|---|---|---|---|
| | | | PCT/JP2020/026595 |

A. CLASSIFICATION OF SUBJECT MATTER
C12M 1/00(2006.01)i; C12M 3/00(2006.01)i
FI: C12M3/00 Z; C12M1/00 D

According to International Patent Classification (IPC) or to both national classification and IPC

B. FIELDS SEARCHED

Minimum documentation searched (classification system followed by classification symbols)
C12M1/00; C12M3/00

Documentation searched other than minimum documentation to the extent that such documents are included in the fields searched
| | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 11-253154 A (KANEGAFUCHI CHEM IND CO., LTD.) 21.09.1999 (1999-09-21) entire text | 1–12 |
| A | JP 2011-628226 A (MILLIPORE CORPORATION) 17.11.2011 (2011-11-17) entire text | 1–12 |
| A | JP 2009-602166 A (CORNING INCORPORATED) 29.01.2009 (2009-01-29) entire text | 1–12 |
| A | WO 2008/106012 A1 (CORNINC INCORPORATED) 04.09.2008 (2008-09-04) entire text | 1–12 |

☐ Further documents are listed in the continuation of Box C.   ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 03 September 2020 (03.09.2020) | 15 September 2020 (15.09.2020) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**
Information on patent family members

International application No.

PCT/JP2020/026595

| Patent Documents referred in the Report | Publication Date | Patent Family | Publication Date |
|---|---|---|---|
| JP 11-253154 A | 21 Sep. 1999 | (Family: none) | |
| JP 2011-528226 A | 17 Nov. 2011 | US 2010/0129900 A1<br>WO 2010/008566 A2<br>EP 2304019 A2<br>CN 102099459 A | |
| JP 2009-502165 A | 29 Jan. 2009 | US 2007/0026516 A1<br>WO 2007/015770 A1<br>EP 1910512 A1 | |
| WO 2008/106012 A1 | 04 Sep. 2008 | JP 2010-518879 A<br>US 2008/0206857 A1<br>EP 2118259 A1 | |

Form PCT/ISA/210 (patent family annex) (January 2015)

**EP 3 998 329 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006262876 A
- JP 2011528226 A
- JP 2009502165 A